# EUROPÄISCHE PATENTSCHRIFT

(11) **EP 3 013 278 B1**
(45) Veröffentlichungstag und Bekanntmachung des Hinweises auf die Patenterteilung: **21.02.2018**
(21) Anmeldenummer: 13739370.8
(22) Anmeldetag: 24.06.2013
(51) Int. Cl.: C04B 41/00, C04B 41/85, C04B 41/50, A61K 6/00, A61K 6/02, C09D 13/00, A61C 13/00, A61C 13/083, C03C 23/00, C04B 111/00, C04B 111/82

(54) **EINFÄRBEN VON PORÖSER KERAMIK**
COLORING POROUS CERAMIC
COLORATION DE CÉRAMIQUE POREUSE

(43) Veröffentlichungstag der Anmeldung: 04.05.2016
(73) Patentinhaber: WDT-Wolz-Dental-Technik GmbH, 55566 Bad Sobernheim (DE)
(72) Erfinder: WOLZ, Stefan, 55666 Bad Sobernheim (DE)
(74) Vertreter: Götz, Georg Alois
(86) Internationale Anmeldenummer: PCT/EP2013/063120
(87) Internationale Veröffentlichungsnummer: WO 2014/206439

(56) Entgegenhaltungen:
- WO-A1-2013/070451
- WO-A2-2004/103303
- WO-A2-2005/105688
- US-A- 2 835 600
- US-A- 5 004 417
- US-A1- 2005 175 552
- Y Maniatis / Chapter 2: "The Emergence of Ceramic Technology and its Evolution as Revealed with the use of Scientific Techniques"; "Chapter 2" In: Andrew J. Shortland; Ian Freestone ; Thilo Rehren: "From Mine to Microscope: Advances in the Study of Ancient Technology", 2009, Oxbow Books, XP055103958, ISBN: 978-1-84-217259-9 Seiten 1-18, Seite 8 - Seite 10

## Beschreibung

Die Erfindung betrifft ein Verfahren zum Färben von gebinderter und/oder entbinderter und/oder angesinterter und/oder durchgängig poröser Keramik, insbesondere von porösen Keramikformkörpern, die insbesondere in der Dentaltechnik Anwendung finden, sowie ein Verbundmaterial zum Auftragen auf die Keramik.

In den letzten Jahren hat sich in der Dentaltechnik im Bereich Vollkeramik hydrostabilisiertes Zirkon durchgesetzt. Der ausschlaggebende Grund dafür ist die hohe Stabilität der Hochleistungskeramikgerüste. Jedoch wurde anhand von Studien nachgewiesen, dass die ästhetische Verblendkeramik, die exzellente Farbschichten und Formgebungsmöglichkeiten besitzt, eine fünfmal höhere Abplatzungsrate als die bewährte Verblendmetallkeramik (VMK) aufweist. Auf Grund der Reklamationen und der hohen Kosten soll auf die ästhetische Verblendkeramik, bei der die Keramikschicht aufwendig mit der Hand aufgebracht wird, verzichtet werden. Ohne diese farbgebenden Möglichkeiten, die die Verblendkeramik besitzt, kann jedoch nur ein Zahnersatz hergestellt werden, der so gut wie keine Ästhetik aufweist.

Gemäß DE 199 04 522 B4, DE 10 2008 026 980 A1, WO 00/46168 A1, WO 2011/156602 A2 oder DE 20 2011 109 956 U1 wurden Metallionen- und/oder Metallkomplexlösungen und Gele entwickelt, die den einfarbigen Grundkörper eines Zahnes imitieren. Jedoch ist anzumerken, dass es keinen einfarbigen Zahn im Mund eines Patienten gibt. Dies wird auch in der DE 10 2008 026 980 A1 erklärt. An den Ecken eines Zahnes, wie zum Beispiel an einer Schneidekante, sammeln sich Farbpigmente, wodurch diese Ecken dunkler als der restliche Zahn wirken. Bei einem natürlichen Zahn wird der farbgebundene dunklere Dentinkern bis hin zu transparenten Zahnschneidekanten von einer immer dicker werdenden Schneidezahnmasse überzogen.

Durch das Eintauchen oder Besprühen des Zahnersatzes wird somit maximal eine einfache und einfarbige Färbung erreicht. Es wird zwar versucht mittels eines Pinsels oder Tropfenapplikationen Zahnfärbungsstrukturen oder ähnliche Einfärbungen auf dem Zahnersatz zu erzielen, jedoch können die bekannten Flüssigkeiten bzw. Lösungen oder Gele, die nur eine Konzentration von Metallionen und/oder Metallkomplexen aufweisen, nicht so auf durchgängig poröser Keramik verteilt werden, dass sich zwischen natürlichen Zähnen und Zahnersatz keine merklich farblichen Unterschiede ergeben, da sie wie beschrieben nur eine bestimmte Konzentration von Metallionen und/oder Metallkomplexen aufweisen. Des Weiteren ist aus der DE 199 04 522 B4 bekannt, dass bei einer Einwirkungsdauer der Flüssigkeit bzw. Lösung oder Gel von 2 Minuten bis 20 Minuten immer der gleiche Farbton erreicht wird. Die Einwirkungstiefe ist außerdem entscheidend für die Farbentwicklung, vor allem beim Übergang in eine andere Farbe.

Ein weiterer Nachteil bei Flüssigkeiten bzw. Lösungen ergibt sich dadurch, dass deren Tropfen nicht lagebestimmt aufgetragen werden kann und sich somit unkontrollierbar auf der durchgängig porösen Keramik verteilt. Es ist zum Beispiel nicht möglich nur die Kronenecken oder nur die Kronenspitzen einzufärben, da der farbgebende Tropfen genau an den Ecken oder Spitzen verläuft.

Ein Pinsel, der zum Auftragen der Farbe genutzt wird, hat zum Beispiel ein unkontrollierbares Depot an Einfärbungsflüssigkeit oder -gel. Es ist demnach ein Zufallsprinzip, wie viel Färbungsmittel der Pinsel aufgenommen hat. Ein Tropfen zuviel kann außerdem nicht vermieden noch korrigiert werden. Falls dies passiert, muss der komplette Zahnersatz neu angefertigt bzw. hergestellt werden.

Aus der US2005/175552 A1 ist ein Verbundmaterial bekannt, welches ein natürliches oder synthetisches Wachs und einen Farbstoff umfasst. Zur Anwendung wird das Verbundmaterial, vorzugsweise in flüssiger Form, auf einen Zahn oder Zahnersatz aufgetragen und bildet an dessen Oberfläche eine Beschichtung bzw. einen Film, welcher dem Zahn oder Zahnersatz eine andere Farbe verleiht. Die WO 2005/105688 A2 offenbart ebenfalls ein Verbundmaterial aus nicht-polaren, chemischen Stoffen sowie mineralischen Elementen. Das Verbundmaterial wird zur Dekoration von Keramiken auf deren Oberfläche aufgetragen, die Keramiken werden anschließend glasiert und gebrannt. Das Verbundmaterial liegt hierzu in der Form von Stiften vor, wodurch ein linienförmiger bzw. zeichnerischer Auftrag ermöglicht wird. Die nicht-polaren chemischen Stoffe verhindern ein ineinander fließen einzelner Linien.

Damit eine Krone bzw. der Zahnersatz ästhetisch wirkt, sollten die im nachfolgenden aufgeführten Merkmale berücksichtigt und umgesetzt werden. Zum einen muss hierfür die Dentinschicht die Grundzahnfarbe des Patienten aufweisen. Die Dentinfarbe und die Schneideschichtstärke bilden die eigentliche Zahnfarbe des Patienten. Zum anderen muss ein immer heller werdender Farbverlauf vom Dentinkern zur Schneide hin erreicht werden. Der Zahnschmelz kann außerdem teilweise helle und/oder transparente Stellen aufweisen. Des Weiteren sind bei den älteren Patienten die dunklen dentinfarbenen Kronenränder gut sichtbar, so dass der neue Zahnersatz dementsprechend angepasst werden muss.

Die Lösungen und Gele, die auf Wasser- und/oder Alkoholbasis aufgebaut sind, unterliegen einer schnellen Verdunstung. Die Verdunstung, die zum Beispiel durch falsche Lagerung entsteht, erhöht die Konzentration der Lösungen und Gele, so dass der Farbton ungewollt verändert wird. Des Weiteren können Lösungen beziehungsweise Flüssigkeiten oder Gele tropfen und auslaufen und somit Verschmutzungen, Verdunstungen und Verklebungen hinterlassen. Demnach ist das Tragen von Schutzkleidung, Schutzhandschuhen und Schutzbrille Vorschrift.

Es ist daher Aufgabe der Erfindung ein Verfahren zum Färben von durchgängig poröser Keramik, insbesondere für die Dentaltechnik, sowie ein Verbundmaterial anzugeben, bei denen die aus dem Stand der Technik geschilderten Nachteile vermieden werden.

Zur Lösung wird zum einen ein Verfahren im Patentanspruch 1 und zum anderen ein Verbundmaterial im Patentanspruch 15 vorgeschlagen. Vorteilhafte, optionale Ausbildungen und/oder Weiterbildungen ergeben sich ganz oder teilweise aus den abhängigen Ansprüchen.

Bei dem vorgeschlagenen Verfahren zum Färben von gebinderter und/oder entbinderter und/oder angesinterter und/oder durchgängig poröser Keramik, insbesondere von porösen Keramikformkörpern, die insbesondere in der Dentaltechnik Anwendung finden, erfolgt der farbgebende Auftrag mit einem Auftragungs- und/oder stiftartigen Werkzeug, das Verbundmaterial, insbesondere Malstiftverbundmaterial, aufweist. Das Verbundmaterial ist oder wird mit farbgebenden Komponenten und/oder brandfesten Pigmenten und/oder Oxiden und/oder färbenden und fluoreszierenden Metalloxiden und/oder organischen oder anorganischen Salzen, insbesondere Metallionen und/oder Metallkomplexe, versetzt. Beispiele für Salze sind Sulfide, Tenside usw. Das Malstiftverbundmaterial ist ein Verbundmaterial, das zum Malen oder Schreiben in einem Stift oder in einem stiftartigen Werkzeug eingebracht ist. Der farbgebende Auftrag kann jedoch mit allen beliebigen beziehungsweise bekannten Auftragungs-Werkzeugen durchgeführt werden. Auftragungs-Werkzeuge beziehungsweise deren entsprechende Verfahren sind beispielsweise: Lichtbogenionenplattierung, Lichtbogen-Friktionsarme-Beschichtung, Drucktechnologien wie zum Beispiel Siebdruck, punktuelle Erwärmung durch Optiken wie zum Beispiel Laser, antistatischer oder elektrostatischer oder elektrographischer oder plasmapolymerisierter Film, Kaltgasspritztechnik, farbgebende Walzen, Pulversprühvorrichtungen, Plasmaentladung und photokatalytische aktive Beschichtung hydrophiler und/oder infiltrierter Schichten. Das bedeutet, dass der farbgebende Auftrag oder die farbgebenden Komponenten durch beispielsweise obig genannte Technologien aufgetragen werden können. Dies stellt eine Art Beschichtung der Oberfläche der porösen Keramik dar, wodurch hydrophile und/oder infiltrierbare Schichten erzeugt werden.

Erfindungsgemäß werden aus dem Verbundmaterial die farbgebenden Komponenten und/oder brandfesten Pigmente und/oder Oxide und/oder färbenden und fluoreszierenden Metalloxide und/oder organischen oder anorganischen Salze mit einem Lösungsmittel herausgelöst, um eine Infiltration herbeizuführen. Das bedeutet, dass das Verbundmaterial bei feuchtem und/oder nassem Untergrund auslösend wirkt. Ein Beispiel für einen feuchten und/oder nassen Untergrund ist eine mit Lösungsmittel getränkte Watte. Eine weitere Möglichkeit ist das Besprühen oder Bedampfen der Keramik zum Beispiel in einem Feuchtigkeitsschrank.

Als Lösungsmittel wird erfindungsgemäß Wasser oder eine Mischung aus Wasser mit einem organischen, insbesondere einem polaren organischen Lösungsmittel verwendet. Die Auslösung der farbgebenden Komponenten und/oder brandfesten Pigmente und/oder Oxide und/oder färbenden und fluoreszierenden Metalloxide und/oder organischen oder anorganischen Salze aus dem Verbundmaterial erfolgt durch eine wässrige Lösung oder Destillat auf Alkoholbasis. Organische Lösungsmittel sind zum Beispiel aliphatische Alkohole. Das Lösungsmittel kann gegebenenfalls Additive, wie zum Beispiel Stabilisatoren oder Elektrolyte, Komplexbildner, Dispergiermittel usw., beinhalten. Die Additive sind entweder im Verbundmaterial oder im Lösungsmittel enthalten.

Es ist außerdem zweckmäßig, dass in das Lösungsmittel ein Oxidationsmittel, insbesondere Wasserstoffperoxid, eingebracht wird. Ebenfalls ist es zweckmäßig, dass in das Lösungsmittel Öle und/oder Benzine eingebracht werden.

Bei einer möglichen erfindungsgemäßen Verfahrensvariante werden die die farbgebenden Komponenten und/oder brandfesten Pigmente und/oder Oxide und/oder färbenden und fluoreszierenden Metalloxide und/oder organischen oder anorganischen Salze in/unter einer Vakuumatmosphäre herausgelöst. Das Herauslösen der farbgebenden Komponenten und/oder brandfesten Pigmente und/oder Oxide und/oder färbenden und fluoreszierenden Metalloxide und/oder organischen oder anorganischen Salze aus dem Verbundmaterial kann auch unter einer annähernden Vakuumatmosphäre stattfinden. Dies ist jedoch nicht zwingend erforderlich.

Bei einer weiteren erfindungsgemäßen Verfahrensvariante werden die farbgebenden Komponenten und/oder brandfesten Pigmente und/oder Oxide und/oder färbenden und fluoreszierenden Metalloxide und/oder organischen oder anorganischen Salze unter Druck herausgelöst. Die farbgebenden Komponenten und/oder brandfesten Pigmente und/oder Oxide und/oder färbenden und fluoreszierenden Metalloxide und/oder organischen oder anorganischen Salze scheiden somit unter Druckbeaufschlagung aus dem Verbundmaterial aus.

Bei dem erfindungsgemäßen Verfahren werden die farbgebenden Komponenten und/oder brandfesten Pigmente und/oder Oxide und/oder färbenden und fluoreszierenden Metalloxide und/oder organischen oder anorganischen Salze in die Poren der porösen Keramik infiltriert und/oder eingebracht. An der Oberfläche der Keramik sind Porenöffnungen vorhanden, so dass die farbgebenden Komponenten und/oder brandfesten Pigmente und/oder Oxide und/oder färbenden und fluoreszierenden Metalloxide und/oder organischen oder anorganischen Salze in diese eindringen. Die Eindringtiefe ist abhängig von der Infiltrationszeit.

Zweckmäßigerweise wird eine Infiltration von farbgebenden Komponenten und/oder brandfesten Pigmenten und/oder Oxiden und/oder färbenden und fluoreszierenden Metalloxiden und/oder organischen oder anorganischen Salzen mittels Kapillarwirkung bewirkt. Das bedeutet, dass auf Grund des Kapillareffekts die die farbgebenden Komponenten und/oder brandfesten Pigmente und/oder Oxide und/oder färbenden und fluoreszierenden Metalloxide und/oder organischen oder anorganischen Salze in die Poren infiltriert werden. Die farbgebenden Komponenten und/oder brandfesten Pigmente und/oder Oxide und/oder färbenden und fluoreszierenden Metalloxide und/oder organischen oder anorganischen Salze werden somit von der Oberfläche der Keramik bis in die Poren der Keramik transportiert. Der Kapillareffekt entsteht aber nur, wenn sich die farbgebenden Komponenten und/oder brandfesten Pigmente und/oder Oxide und/oder färbenden und fluoreszierenden Metalloxide und/oder organischen oder anorganischen Salze in einer Flüssigkeit oder in einer Lösung befinden. Dies geschieht durch die Verwendung von einem wässrigen Lösungsmittel oder bei Einbringen von Wärme, so dass das Verbundmaterial schmilzt und eine Flüssigkeit darstellt.

Das erfindungsgemäße Verfahren bietet außerdem die Möglichkeit, dass ein oder mehrere Verbundmaterialen nacheinander auf die poröse Keramik aufgetragen und/oder teilweise abgetragen und gegebenenfalls anschließend wieder aufgetragen werden. Alle Verbundmaterialien, die auf der Keramik aufgetragen werden, verhalten sich passiv. Das bedeutet, dass keine Infiltration, d.h. keine Kapillarwirkung ohne einen zusätzlichen Schritt stattfindet. Das heißt, dass das auf der Oberfläche aufgetragene Verbundmaterial nicht ohne zusätzliche Schritte mit der Keramik reagiert und somit zu jeder Zeit mit anderen Verbundmaterialien und/oder Verbundmaterialkonzentrationen vermischt werden kann. Das hat ebenfalls den Vorteil, dass zu viel aufgetragenes Verbundmaterial ohne Probleme wieder entfernt werden kann. Des Weiteren kann zuviel aufgetragenes oder falsches farbgebendes Verbundmaterial wieder abgetragen werden. Anschließend besteht die Möglichkeit ein gewünschtes Verbundmaterial, d.h. entweder das gleiche oder ein anderes, auf die Keramik aufzutragen.

Während des erfindungsgemäßen Verfahrens ist es vorteilhaft, wenn Farbmessungen an natürlichen Zähnen in einem programmierbaren Speicher hinterlegt werden, so dass die Farbe der natürlichen Zähne mit dem entsprechenden Verbundmaterial reproduziert werden kann. Die Zahnfarbe der natürlichen Zähne des Patienten wird demnach gemessen. Anschließend werden die ermittelten Farbdaten in einer Software oder in einem programmierbaren Speicher gespeichert. Die Software oder der programmierbare Speicher kann mittels der Farbdaten die natürliche Zahnfarbe mit den Verbundmaterialien berechnen bzw. reproduzieren.

Des Weiteren ist es zweckmäßig, dass während des Verfahrens ein oder mehrere Applikationsinstrumente zum Auftrag eines oder mehrerer Verbundmaterialien verwendet werden. Zum Beispiel wird das Verbundmaterial von Auftragungs- und/oder stiftartigen Werkzeugen umschlossen bzw. umgeben. Das bedeutet, dass als Applikationsinstrument auch ein handelsüblicher Malstift verwendet werden kann, in dem statt der Farbe das Verbundmaterial vorgesehen ist. Es ist jedoch auch möglich alle bekannten beziehungsweise im Stand der Technik vorkommenden Auftragungs-Werkzeuge hierfür zu nutzen.

Eine weitere Möglichkeit des Verfahrens der Erfindung ist, dass eine programmgesteuerte Maschine oder Gerät, insbesondere eine CAD/CAM-Anlage, zum Auftrag oder zum Abtrag eines oder mehrerer Verbundmaterialien entsprechend der festgelegten Zahnfarbe verwendet wird. Die CAD/CAM-Anlage beziehungsweise die programmgesteuerte Maschine oder Gerät besitzt zum Beispiel ein Magazin mit Verbundmaterialien, die jeweils unterschiedliche Konzentrationen aufweisen. Nach dem die Implantatprothetik oder Implantate und/oder der Zahnersatz, insbesondere Kronen und Brücken, von der CAD/CAM-Anlage beziehungsweise von der programmgesteuerten Maschine oder Gerät angefertigt wurde, wird anstelle eines weiteren Fräsers das gewünschte Verbundmaterial aus dem Magazin genommen. Des Weiteren kann eine weitere oder die gleiche programmgesteuerte Maschine einen Farbkontrollscan durchführen bevor die farbgebenden Komponenten und/oder brandfesten Pigmente und/oder Oxide und/oder färbenden und fluoreszierenden Metalloxide und/oder organischen oder anorganischen Salze in die poröse Keramik infiltriert werden oder bevor die bereits infiltrierte Keramik gesintert wird. Dadurch kann gegebenenfalls der Farbton korrigiert werden beziehungsweise eine hohe Farbgenauigkeit erreicht werden.

Es werden des Weiteren erfindungsgemäß die Farbdaten der durchgeführten Farbmessung an die programmgesteuerte Maschine oder an das programmgesteuerte Gerät übermittelt. Die zuvor ermittelten Farbdaten der natürlichen Zähne des Patienten werden an die programmgesteuerte Maschine oder Gerät übermittelt, so dass die Maschine ein oder mehrere Verbundmaterialien verwendet, die der Zahnfarbe der natürlichen Zähne des Patienten entsprechen.

Bei einer vorteilhaften Verfahrensvariante werden ganze vorgesinterte und/oder vorgepresste Keramikblöcke vor der CAD/CAM Bearbeitung eingefärbt. Das bedeutet, dass Keramikblöcke bereits in unterschiedlichen Grundfarben hergestellt werden können, damit nur noch die Abstufungen oder die dunkleren gewünschten Farben, zum Beispiel an den Randzonen, aufgetragen werden müssen.

Bei dem erfindungsgemäßen Verfahren wird der farbgebende Auftrag oder Verbundmaterial an den Außen- und/oder Innenflächen des Zahnersatzes und/oder der Implantatprothetik oder der Implantate aufgetragen und/oder abgetragen. Ob an den Außenflächen oder an den Innenflächen das jeweilige Verbundmaterial aufgetragen und/oder abgetragen wird, ist abhängig von der gewünschten Farbe beziehungsweise der gewünschten Tiefenwirkung.

Ein weiterer beispielhafter Schritt hinsichtlich des erfindungsgemäßen Verfahrens ist es, dass die Rückstände des Verbundmaterials nach der Infiltration rückstandslos verbrannt werden oder mittels einer Lösung entfernt werden. Der wichtigste Bestandteil des Verbundmaterials bilden die farbgebenden Komponenten und/oder brandfesten Pigmente und/oder Oxide und/oder färbenden und fluoreszierenden Metalloxide und/oder organischen oder anorganischen Salze. Die restlichen Bestandteile oder Füllstoffe des Verbundmaterials können entweder verbrannt oder mit einem Lösungsmittel entfernt werden. Es müssen nach der Infiltration alle Rückstände des aufgetragenen Verbundmaterials entfernt werden können, da diese Rückstände während dem Sintern nicht gewünschte Oxidationen hervorrufen können.

Durch das erfindungsgemäße Verfahren wird ein glasurartiger Verschluss der Porosität der einzufärbenden Keramik durch das aufgetragene Verbundmaterial erzeugt. Es werden somit also die Poren der porösen Keramik verschlossen.

Für eine Verfahrensausführungsform wird eine porös gebrannte Dentalverblendkeramik oder eine porös gepresste Glaskeramik als zu bemalende Keramik verwendet. Das Verfahren ist bei allen porösen Keramiken anwendbar. Unabhängig ob es sich dabei um eine gepresste, gebrannte, gebinderte, entbinderte und/oder angesinterte Keramik handelt. Die Keramik muss jedoch Poren aufweisen, so dass die farbgebenden Komponenten und/oder brandfesten Pigmente und/oder Oxide und/oder färbenden und fluoreszierenden Metalloxide und/oder organischen oder anorganischen Salze in diese infiltrieren können.

Bei dem Verfahren wird beispielsweise ein Verbundmaterial, das Zirkonoxid (ZrO₂), Yttriumoxid (Y₂O₃), Hafniumoxid (HfO₇), Aluminiumoxid (Al₂O₃) Phosphoroxid (P₂O₃, P₂O₄ P₄O₁₀), Titanoxid (TiO₂), Zinnoxid (SnO, Sn₂O₃, SnO₂), Boroxid ((BO)ₓ, (B₂O)ₓ), Bortrioxid (B₂O₃), Fluor (F₂), Natriumoxid (Na₂O), Bariumoxid (BaO), Strontiumoxid (SrO), Strontiumperoxid (SrO₂), Magnesiumoxid (MgO), Zinkoxid (ZnO), Zinnoxid (SnO, Sn₂O₃, SnO₂), Calciumoxid (CaO), Titanoxid (TiO₂), Nioboxid (NbO, NbO₂, Nb₂O₅ Tantaloxid (TaO, TaO₂, Ta₂O₅), Lanthanoxid (La₂O₃), Siliciumdioxid (SiO₂), Leucit, Vanadium(V)-oxid (V₂O₅), dotierte Spinelle und/oder weitere Oxide sowie Mischungen hiervon umfasst, verwendet.

Des Weiteren kann bei dem erfindungsgemäßen Verfahren Feldspatkeramik, Zirkonoxid- und/oder Leucitverstärkte-Keramik, Lithiumsilikat-Gläser oder Lithiumsilikat-Glaskeramik oder Lithiumdisilikat-Glaskeramik, Silikatkeramik und/oder Oxidkeramik eingefärbt werden. Als einzufärbende Keramik für das Verfahren kann jede Oxidkeramik oder eine Keramik auf Basis von Oxidkeramik verwendet werden. Oxidkeramiken sind Hochleistungskeramiken, die härter, verschleißfester, wärmebeständiger und spröder als Hartmetalle sind. Sie bieten demnach die wichtigsten Eigenschaften für eine Implantatprothetik oder ein Implantat und/oder einen Zahnersatz. Des Weiteren kann auch eine Keramik, die auf Basis von Glaskeramik und/oder Glas besteht, verwendet werden.

Bei einem erfindungsgemäßen Verfahrensschritt wird das auf der Keramik aufgetragene und infiltrierte Verbundmaterial unter ansteigender Vakuumatmosphäre oder in einem sauerstofffreien oder annähernd sauerstofffreien Raum gesintert, um die farbgebenden Komponeten und/oder brandfesten Pigemente und/oder Oxide und/oder färbenden und fluoreszierenden Metalloxide und/oder organischen oder anorganischen Salze in der Keramik zu fixieren. Das bedeutet, dass die farbgebenden Komponeten und/oder brandfesten Pigemente und/oder Oxide und/oder färbenden und fluoreszierenden Metalloxide und/oder organischen oder anorganischen Salze in den Poren der Keramik durch einen Sintervorgang fixiert werden.

Bei einem erfindungsgemäßen Ausführungsverfahren wird das Verbundmaterial mit Opaker- und/oder Glaslot-Komponenten und/oder - Pigmenten und/oder keramischen Komponenten und/oder -Pigmenten versetzt. Ein Beispiel für keramische Komponenten und/oder -Pigmente ist SiO₂-basiertes Spezialglas mit minimaler Leucitphase. Damit kann zum Beispiel Titan mit Zirkonoxid verbunden werden. Des Weiteren wird erfindungsgemäß nach dem Auftragen des Verbundmaterials auf die Keramik ein Metallelement und/oder ein Keramikelement aufgebracht. Bei einem weiteren erfindungsgemäßen Verfahrensschritt wird mittels sintern die infiltrierte Keramik eingefärbt und mit dem Metallelement und/oder mit dem Keramikelement verbunden. Dadurch entsteht zwischen dem Metallelement, insbesondere Metallgerüst, und/oder dem Keramikelement und dem Keramikkörper ein Haftverbund. Dieser Haftverbund wird durch mindestens einen Sintervorgang hergestellt. Der Haftverbund deckt außerdem die Farbe des Metallelements ab.

Das erfindungsgemäße Verfahren umfasst folgende Schritte:
(a) Bereitstellen einer durchgängig porösen und/oder vorgesinterten und/oder entbinderten und/oder gebinderten Keramik, insbesondere eines Keramikformkörpers
(b) und Behandlung der Keramik mit mindestens einem Verbundmaterial, das farbgebende Komponenten und/oder brandfeste Pigmente und/oder Oxide und/oder färbende und fluoreszierende Metalloxide und/oder organische oder anorganische Salze beinhaltet
(c) und Auslösung der farbgebenden Komponenten und/oder brandfesten Pigmente und/oder Oxide und/oder färbenden und fluoreszierenden Metalloxide und/oder organischen oder anorganischen Salze aus dem Verbundmaterial mittels eines Lösungsmittels und/oder mittels einer Wärmebehandlung, wodurch die Infiltration der farbgebenden Komponenten und/oder brandfesten Pigmente und/oder Oxide und/oder färbenden und fluoreszierenden Metalloxide und/oder organischen oder anorganischen Salze in die Poren der bemalten Keramik ausgelöst wird
(d) und gegebenenfalls Trocknung der behandelten Keramik, insbesondere nach Verwenden eines Lösungsmittels, bei einer Temperatur unterhalb der Sintertemperatur oder des Siedepunkts, insbesondere unterhalb von 100°C
(e) und/oder Sintern der Keramik, um die farbgebenden Komponenten und/oder brandfesten Pigmente und/oder Oxide und/oder färbenden und fluoreszierenden Metalloxide und/oder organischen oder anorganischen Salze in den Poren zu fixieren.

Neben dem Verfahren wird auch ein erfindungsgemäßes Verbundmaterial vorgeschlagen. Das Verbundmaterial, insbesondere geeignet für Malstifte, zum Auftragen auf durchgängig poröse Keramik, weist farbgebende Komponenten und/oder brandfeste Pigmente und/oder Oxide und/oder färbende und fluoreszierende Metalloxide und/oder organische oder anorganische Salze und einen oder mehrere Füllstoffe auf. Die Kombination der farbgebenden Komponenten und/oder brandfesten Pigmente und/oder Oxide und/oder färbenden und fluoreszierenden Metalloxide und/oder organischen oder anorganischen Salze und des/der Füllstoffe stellt ein Färbemittel, insbesondere für die Zahntechnik, dar, wobei das Färbemittel unterschiedliche Konsistenzen aufweist. Es können alle möglichen beziehungsweise erdenklichen Konsistenzen des Verbundmaterials hergestellt werden. Das bedeutet, dass die Konsistenzen der Verbundmaterialien von weich bis sehr hart reichen. Zum Beispiel ist unter weicher Konsistenz eine Paste zu verstehen. Die Paste ist mit einer Kugelschreiberpaste zu vergleichen. Mittel-harte Konsistenz kann zum Beispiel unter der Lippenstiftkonsistenz verstanden werden. Unter harter Konsistenz wird zum Beispiel die Konsistenz eines Wachsmalstifts verstanden. Bei sehr harter Konsistenz ist zum Beispiel eine Bleistiftmiene zu verstehen. Es können somit alle Konsistenzen, die derzeit bekannt sind, für die Konsistenz des Verbundmaterials verwendet werden. Die Konsistenzen sollten jedoch nicht flüssig und/oder wässrig sein, so dass die Infiltration sofort nach dem Auftragen stattfindet ohne einen zusätzlichen Schritt durchzuführen.

In dem erfindungsgemäßen Verbundmaterial sind Konzentrationen der farbgebenden Komponenten und/oder brandfesten Pigmente und/oder Oxide und/oder färbenden und fluoreszierenden Metalloxide und/oder organischen oder anorganischen Salze zwischen 0,001 Gew.-% und 80 Gew.-% oder zwischen 0,005 Gew.-% und 60 Gew.-% enthalten. Je nachdem welche Farbe erreicht werden soll, sind entweder geringe oder große Mengen an farbgebenden Komponenten und/oder brandfesten Pigmenten und/oder Oxiden und/oder färbenden und fluoreszierenden Metalloxiden und/oder organischen oder anorganischen Salzen erforderlich. Demnach sind die Konzentrationen der farbgebenden Komponenten und/oder brandfesten Pigmente und/oder Oxide und/oder färbenden und fluoreszierenden Metalloxide und/oder organischen oder anorganischen Salze in dem Verbundmaterial an die gewünschte Farbe anzupassen.

Es ist außerdem zweckmäßig, dass ein Füllstoff des erfindungsgemäßen Verbundmaterials oxidkeramisches und/oder glaskeramisches Material beinhaltet.

Des Weiteren ist es vorteilhaft, wenn ein Füllstoff Lipide, vorzugsweise Fette oder Wachse, beinhaltet. Das bedeutet, dass das Verbundmaterial gegebenenfalls Lipide beinhaltet. Die Lipide schmelzen bei geringer beziehungsweise langsamer Wärmeeinbringung und verbrennen beim Erhitzen.

Erfindungsgemäß ist das Färbemittel wasserlöslich. Das bedeutet, dass die Kombination aus den farbgebenden Komponenten und/oder brandfesten Pigmenten und/oder Oxiden und/oder färbenden und fluoreszierenden Metalloxiden und/oder organischen oder anorganischen Salzen und den Füllstoffen wasserlöslich ist. Demnach kann das aufgetragene Färbemittel oder Verbundmaterial mittels einer wässrigen Lösung entfernt werden. Unter wässriger Lösung werden alle Lösungen beziehungsweise Flüssigkeiten verstanden, wie zum Beispiel Wasser, Alkohole und/oder Öle.

Außerdem ist es zweckmäßig, dass die Füllstoffe mit Farbpigmenten versehen sind, die auf die Konzentration der im Verbundmaterial enthaltenen farbgebenden Komponenten und/oder brandfesten Pigmenten und/oder Oxiden und/oder färbenden und fluoreszierenden Metalloxiden und/oder organischen oder anorganischen Salzen hinweisen. Das bedeutet, dass das Verbundmaterial eine bestimmte Farbe aufweist, die einer bestimmten Konzentration der farbgebenden Komponenten und/oder brandfesten Pigmenten und/oder Oxiden und/oder färbenden und fluoreszierenden Metalloxiden und/oder organischen oder anorganischen Salzen zugeordnet ist. Die Farbe oder die Farbpigmente werden in den Füllstoffen beigemischt.

Die Zusammensetzung des Verbundmaterials entspricht beispielsweise erfindungsgemäß handelsüblichen Wassermalstiften.

Ein erfindungsgemäßes Ausführungsbeispiel stellt ein Verbundmaterial dar, wobei die farbgebenden Komponenten und/oder brandfesten Pigmente und/oder Oxide und/oder färbenden und fluoreszierenden Metalloxide und/oder organischen oder anorganischen Salze Seltenerdmetalle, insbesondere Lanthanoide, Neodym, Paseodym, Samarium, Europium oder chemische Elemente der 3. Nebengruppe des Periodensystems, beinhalten. Zu den Seltenerdmetallen zählen die chemischen Elemente der 3. Nebengruppe des Periodensystems und die Lanthanoide. Das bedeutet, dass zu den Seltenerdmetallen 17 Elemente gehören, wobei ein oder mehrere Elemente eventuell in den farbgebenden Komponenten und/oder brandfesten Pigmenten und/oder Oxiden und/oder färbenden und fluoreszierenden Metalloxiden und/oder organischen oder anorganischen Salzen enthalten sind. Dem Fachmann sind dadurch alle weiteren Möglichkeiten bekannt.

Ein weiteres erfindungsgemäßes Ausführungsbeispiel stellt ein Verbundmaterial dar, wobei die farbgebenden Komponenten und/oder brandfesten Pigmente und/oder Oxide und/oder färbenden und fluoreszierenden Metalloxide und/oder organischen oder anorganischen Salze mindestens eines der Elemente Yttrium, Eisen, Titan, Selen, Silber, Indium, Gold, Chrom, Kupfer, Praseodym, Kobalt, Nickel, Mangan, Erbium, Cer oder Seltenerdmetalle sowie Mischungen hiervon beinhalten. Die in das Verbundmaterial eingebrachten farbgebenden Komponenten und/oder brandfesten Pigmente und/oder Oxide und/oder färbenden und fluoreszierenden Metalloxide und/oder organischen oder anorganischen Salze weisen somit eines oder mehrere Elemente wie Yttrium, Eisen, Titan, Selen, Silber, Indium, Gold, Chrom, Kupfer, Praseodym, Kobalt, Nickel, Mangan, Erbium, Cer oder Seltenerdmetalle sowie Mischungen hiervon auf.

Des Weiteren beträgt die aufgetragene Schichtstärke des erfindungsgemäßen Verbundmaterials zwischen 0,00001 mm und 1,5 mm. Die Schichtstärke variiert zwischen dem oben genannten Bereich abhängig von der Konsistenz des Verbundmaterials und/oder abhängig vom verwendeten Füllstoff.

Es ist ebenfalls zweckmäßig, dass das Verbundmaterial mit den darin beinhalteten Füllstoffen und farbgebenden Komponenten und/oder brandfesten Pigmenten und/oder Oxiden und/oder färbenden und fluoreszierenden Metalloxiden und/oder organischen oder anorganischen Salzen als Folie adaptierbar ist. Das bedeutet, dass das Verbundmaterial auf einer Folie vorliegen kann oder selbst eine Folie ist. Die Folie kann auf den Zahnersatz und/oder Implantatprothetik oder Implantat oder auf dem Block aufgebracht werden und mittels eines Lösungsmittels von der Folie gelöst werden.

Des Weiteren sind erfindungsgemäß ein oder mehrere Partikelgrößen der Füllstoffe größer als ein oder mehrere Porenöffnungen der Keramik. Das bedeutet, dass die Füllstoffe beziehungsweise Partikel der Füllstoffe nicht in die Poren infiltrieren können, sondern an der Oberfläche der Keramik verbleiben. Das Verbundmaterial beinhaltet demnach unterschiedliche Partikelgrößen, die entweder nicht in die Poren eindringen oder die infiltrationsfähig sind, wie zum Beispiel die farbgebenden Komponenten. Es ist außerdem durchaus möglich, dass der Füllstoff große und kleine Partikel aufweist, wodurch die einen infiltrieren und die anderen auf der Oberfläche der Keramik verbleiben.

Die Füllstoffe beinhalten Nanopartikel, wobei die Nanopartikel eine hydrophile Ligandenbeschichtung aufweisen. Bei einem weiteren erfindungsgemäßen Ausführungsbeispiel beinhaltet ein Füllstoff des Verbundmaterials, insbesondere glashaltiges Material, antimikrobakteriell wirkende Zusätze und/oder Material. Es können auch die antimikrobakteriellen Zusätze und/oder Materialien in keramischen Füllstoffen eingebracht werden. Als antimikrobakterielle Zusätze und/oder Metall, wie zum Beispiel Kupfer oder Silber usw., werden vor allem wasserbeständige Zusätze verstanden. Dadurch können keinerlei Bakterien, Plaque, usw. auf der mit Verbundmaterial behandelten und/oder eingefärbten Keramik auftreten.

Erfindungsgemäß beinhaltet ein Füllstoff des Verbundmaterials Opaker- und/oder Glaslot-Komponenten und/oder -Pigmente. Durch diese Opaker- und/oder Glaslot-Komponenten und/oder -Pigmente wird ein Metallelement, insbesondere ein Metallgerüst, das Edelmetalllegierungen und/oder Nichtedelmetalllegierungen, insbesondere CrCo oder Titan, enthält, mit dem Zahnersatz und/oder Implantatprothetik oder Implantat stoffschlüssig verbunden. Das mit Opaker- und/oder Glaslot-Komponenten und/oder -Pigmenten versetzte Verbundmaterial sollte nur an den Oberflächen aufgetragen werden, an denen anschließend ein Metallelement angebracht wird beziehungsweise angebracht werden soll, da die Opaker- und/oder Glaslot-Komponenten und/oder -Pigmente sehr deckend/abdeckend wirken. Der WAK-Wert ist den zu verwendenden Materialien entsprechend anzupassen.

Ein weiteres vorteilhaftes erfindungsgemäßes Ausführungsbeispiel des Verbundmaterials ist, dass ein Füllstoff keramische Komponenten und/oder -Pigmente beinhaltet. Durch diese keramischen Komponenten und/oder -Pigmente, wie zum Beispiel SiO₂-basiertes Spezialglas, wird ein Keramikelement, insbesondere bestehend aus Zirkonoxid, mit der porösen Keramik, wie zum Beispiel Lithiumsilikat-Glaskeramik und/oder Zirkonoxid und/oder Feldspat, stoffschlüssig miteinander verbunden. Die dadurch entstandene Verbindung von mehreren Keramikschichten stellt den Zahnersatz und/oder Implantatprothetik oder Implantat dar. Eine weitere Alternative mehrere Keramikschichten miteinander zu verbinden liegt darin einen 2-Komponenten-Kleber zu verwenden.

Es werden nicht alle chemischen Verbindungen beziehungsweise Stoffe aufgeführt, die in dem erfindungsgemäßen Verfahren beziehungsweise in dem erfindungsgemäßen Verbundmaterial verwendet werden können. Dem Fachmann sollten jedoch durch die obigen Ausführungen alle Möglichkeiten bekannt sein.

Des Weiteren ist es möglich zuerst eine Lösung oder ein Lösungsmittel aufzutragen beziehungsweise die Keramik damit zu beaufschlagen und erst anschließend ein Verbundmaterial auf der Keramik aufzutragen. Dadurch würde jedoch gleich während dem Auftrag die Infiltration stattfinden, sodass die oben aufgeführten Vorteile wiederum geschmälert werden.

Weitere Einzelheiten, Merkmale, Merkmalskombinationen und Wirkungen auf der Basis der Erfindung ergeben sich aus der nachfolgenden Beschreibung bevorzugter, beispielhafter Ausführungsformen der Erfindung sowie aus den Zeichnungen. Diese zeigen in:
- Figur 1: Ablaufschema des erfindungsgemäßen Verfahrens
- Figur 2: Querschnitt eines Zahnersatzes mit Einfärbungen
- Figur 3: Querschnitt eines Zahnersatzes mit einem aufgebrachten Metallgerüst
- Figur 4: Querschnitt eines eingefärbten Blocks vor der CAD/CAM-Bearbeitung

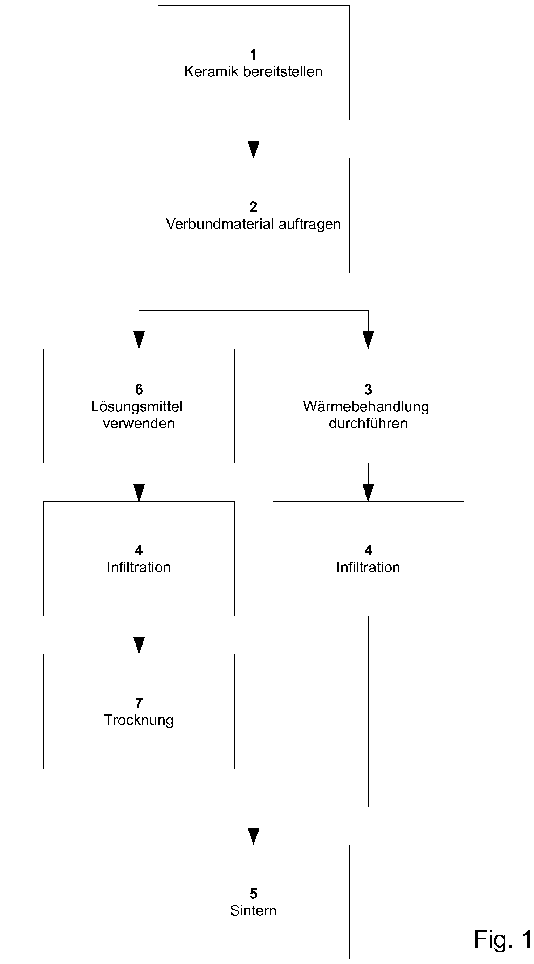

Die Fig. 1 stellt ein Ablaufschema des erfindungsgemäßen Verfahrens dar. Zuerst wird eine poröse und/oder vorgesinterte und/oder entbinderte und/oder gebinderte Keramik bereitgestellt 1. Die Keramik stellt einen Keramikformkörper beziehungsweise einen Zahnersatz und/oder Implantatprothetik oder ein Implantat dar, wie zum Beispiel eine Krone. Anschließend wird das farbgebende Verbundmaterial auf den Keramikformkörper oder Zahnersatz und/oder Implantatprothetik oder Implantat beziehungsweise auf die poröse Keramik aufgetragen **2.** Das Verbundmaterial oder die Verbundmaterialien wird/werden beispielsweise mittels eines Auftragungs- und/oder stiftartigen Werkzeuges auf den Keramikformkörper oder Zahnersatz und/oder Implantatprothetik oder Implantat aufgebracht. Die Keramik wird somit mit den Verbundmaterialien behandelt.

Das bedeutet, dass zunächst auf die poröse Keramik mindestens ein wasserlösliches Verbundmaterial aufgetragen wird. Das Verbundmaterial weist dabei eine beliebige Konzentration von farbgebenden Komponenten und/oder brandfesten Pigmente und/oder Oxide und/oder färbenden und fluoreszierenden Metalloxide und/oder organischen oder anorganischen Salze auf. Abhängig von der Konzentration der farbgebenden Komponenten und/oder brandfesten Pigmenten und/oder Oxiden und/oder färbenden und fluoreszierenden Metalloxiden und/oder organischen oder anorganischen Salzen sind in dem Verbundmaterial beziehungsweise im Füllstoff bestimmte Farbpigmente oder Farben eingebracht, so dass Rückschlüsse auf die Konzentration gemacht werden kann. Das Verbundmaterial wird an den Innen- und/oder Außenflächen des Zahnersatzes und/oder Implantatprothetik oder Implantats aufgetragen. Um die Konzentration lokal zu erhöhen, wie zum Beispiel am Kronenrand, wird das Verbundmaterial einfach mehrmals auf der gleichen Stelle aufgetragen. Die intensivere Farbgebung wird dadurch sichtbar, so dass die Farbe gezielt aufgetragen werden kann. Eine andere Möglichkeit besteht darin, ein Verbundmaterial mit einer bereits erhöhten Konzentration von farbgebenden Komponenten und/oder brandfesten Pigmenten und/oder Oxiden und/oder färbenden und fluoreszierenden Metalloxiden und/oder organischen oder anorganischen Salzen zu verwenden. Nach dem Auftragen oder Abtragen der Verbundmaterialien mit unterschiedlichen Konzentrationen können diese verwischt werden mit zum Beispiel einem Finger oder einem Pinsel, wenn keine farbintensiven Übergänge gewünscht werden oder abgefräst beziehungsweise abgetragen werden bei gewünschten farbintensiven Übergängen.

Das Verbundmaterial weist unterschiedliche Konsistenzen auf. Unter weiches Verbundmaterial wird eine Paste verstanden, wie zum Beispiel eine Kugelschreiberpaste oder MakeUp Creme. Die nächste Abstufung der Konsistenz stellt die mittel-weiche beziehungsweise mittel-harte Konsistenz dar, wie zum Beispiel ein Lippenstift. Unter harter Konsistenz oder Verbundmaterial wird zum Beispiel eine Wachsmalstift-Konsistenz verstanden. Des Weiteren gibt es noch die Möglichkeit eine sehr harte Konsistenz des Verbundmaterials zu nutzen. Die Härte ist mit einer Bleistiftmiene zu vergleichen.

Abhängig von den Füllstoffen des Verbundmaterials unterscheiden sich die nachfolgenden Schritte. Bei Lipiden oder fett- oder wachshaltigen Füllstoffen wird nach dem Auftragen eine Wärmebehandlung durchgeführt **3.** Durch die Wärmeeinbringung werden die farbgebenden Komponenten und/oder brandfesten Pigmente und/oder Oxide und/oder färbenden und fluoreszierenden Metalloxide und/oder organischen oder anorganischen Salze aus dem Verbundmaterial herausgelöst **3,** da die fett- oder wachshaltigen Füllstoffe schmelzen. Durch den Kapillareffekt werden die farbgebenden Komponenten und/oder brandfesten Pigmente und/oder Oxide und/oder färbenden und fluoreszierenden Metalloxide und/oder organischen oder anorganischen Salze in die Poren der Keramik infiltriert, d.h. es findet eine Infiltration der farbgebenden Komponenten und/oder brandfesten Pigmente und/oder Oxide und/oder färbenden und fluoreszierenden Metalloxide und/oder organischen oder anorganischen Salze in den Poren der Keramik statt **4.** Nachdem sich die farbgebenden Komponenten und/oder brandfesten Pigmente und/oder Oxide und/oder färbenden und fluoreszierenden Metalloxide und/oder organischen oder anorganischen Salze in der gewünschten Tiefe der Poren befinden, wird die behandelte Keramik in einem Sinterofen gesintert **5.** Auf Grund der hohen Wärmeeinbringung werden die Poren der porösen Keramik, in denen sich die farbgebenden Komponenten und/oder brandfesten Pigmente und/oder Oxide und/oder färbenden und fluoreszierenden Metalloxide und/oder organischen oder anorganischen Salze befinden, geschlossen beziehungsweise wird die Keramik verdichtet. Die Rückstände des Verbundmaterials werden außerdem durch die hohen Temperaturen beim Sintern rückstandslos verbrannt.

Bei Verwendung eines Verbundmaterials ohne fett- oder wachshaltiger Füllstoffe, wird nach dem Auftragen des Verbundmaterials auf die poröse Keramik **2** ein Lösungsmittel verwendet, um die farbgebenden Komponenten und/oder brandfesten Pigmente und/oder Oxide und/oder färbenden und fluoreszierenden Metalloxide und/oder organischen oder anorganischen Salze aus dem Verbundmaterial herauszulösen **6.** Die poröse Keramik kann zum Beispiel auf eine mit Lösungsmittel getränkte Watte oder in einen Feuchtigkeitsschrank gelegt werden. Die in dem Verbundmaterial lokal konzentrierten farbgebenden Komponenten und/oder brandfesten Pigmente und/oder Oxide und/oder färbenden und fluoreszierenden Metalloxide und/oder organischen oder anorganischen Salze werden durch die wässrige Lösung aus dem Verbundmaterial herausgelöst und anschließend durch die Kapillarwirkung in die durchgängig poröse Keramik infiltriert **4.** Die Infiltrationsgeschwindigkeit liegt bei ca. 0,001mm pro Minute. Anschließend wird die behandelte Keramik, vorzugsweise in einem Trockenschrank, getrocknet, d.h. es findet eine Trocknung der behandelten Keramik statt **7,** so dass die farbgebenden Komponenten und/oder brandfesten Pigmente und/oder Oxide und/oder färbenden und fluoreszierenden Metalloxide und/oder organischen oder anorganischen Salze in der gewünschten Tiefe in den Poren verbleiben. Es besteht jedoch auch die Möglichkeit, dass der Trocknungsschritt nicht durchgeführt werden muss und dieser somit übersprungen wird. Die Trocknung ist zum Beispiel bei Verwendung eines Lösungsmittels, das Öle oder Benzine aufweist, nicht notwendig und kann demnach weggelassen werden. Das bedeutet, dass eventuell direkt nach dem Schritt **4** der Schritt **5** durchgeführt werden kann. Entweder direkt nach der Infiltration **4** oder nach dem Trocknungsschritt **7** wird die Keramik in einem Sinterofen gesintert **5,** so dass eine Färbung der behandelten Keramik entsteht.

Die Gestaltung beziehungsweise Gestaltgebung der Implantatprothetik oder der Implantate und/oder der Zahnersatze beziehungsweise die CAD/CAM-Bearbeitung kann entweder vor oder nach der Infiltration oder vor oder nach dem Sintervorgang stattfinden.

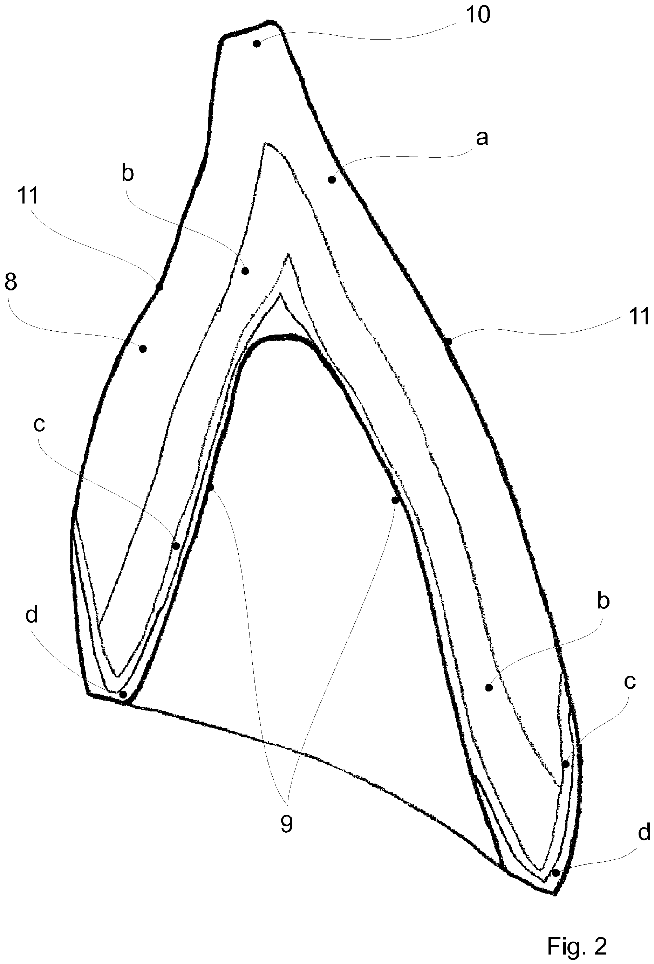

In der Fig. 2 ist ein Querschnitt eines Zahnersatzes **8** mit gewünschten Einfärbungen **a, b, c, d** dargestellt. Vor allem an den Innenflächen **9** des Zahnersatzes **8** sollte die aufgetragene Farbe **d** intensiver sein. Die Schneide **10** des Zahnersatzes **8** hingegen sollte immer heller **a** sein, um eine ästhetische Farbgebung beziehungsweise eine natürliche Farbgebung zu erreichen. An den Außenflächen **11** des Zahnersatzes **8** hingegen sollte die Farbe sehr hell **a** sein. Die natürliche Einfärbung des Zahnersatzes **8** wird erreicht durch Verwendung von mehreren Verbundmaterialien, die jeweils unterschiedliche Konzentrationen der farbgebenden Komponenten und/oder brandfesten Pigmente und/oder Oxide und/oder färbenden und fluoreszierenden Metalloxide und/oder organischen oder anorganischen Salze besitzen. Dabei ist noch anzumerken, dass die Übergänge der unterschiedlichen Einfärbungen fließend sind und nicht unbedingt harte Kanten entstehen müssen. Die in Fig. 2 dargestellten Kanten sind nur zum Verständnis eingezeichnet und existieren bei der Anwendung des oben genannten Verfahrens beziehungsweise des Verbundmaterials nicht, falls dies nicht erwünscht ist.

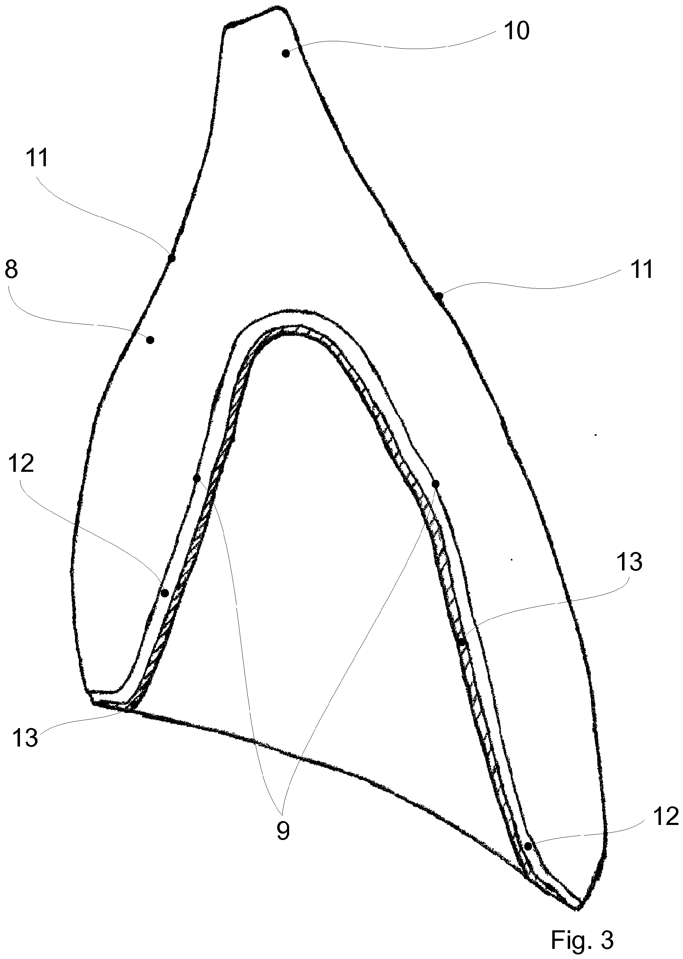

In der Fig. 3 ist ein Querschnitt eines Zahnersatzes **8** dargestellt. An den Innenflächen **9** des Zahnersatzes **8** wird ein farbgebender Auftrag **12,** insbesondere ein Verbundmaterial, das mit Opaker-Komponenten und/oder -Pigmente versehen ist, aufgetragen. Auf diesem farbgebenden Auftrag **12** ist ein Metallgerüst **13** und/oder Metallelement aufgebracht. Dabei ist es wichtig, dass das mit Opaker-Komponenten und/oder -Pigmente versetzte Verbundmaterial nur auf Oberflächen des Zahnersatzes und/oder Implantatprothetik oder Implantat aufgetragen werden sollte, die mit Metall in Verbindung kommen. Mittels den im Verbundmaterial **12** enthaltenen Opaker-Komponenten und/oder -Pigmente wird bei Durchführung des beschriebenen Verfahrens, insbesondere aufgrund des Verfahrensschritts Sintern, das Metallgerüst **13** und/oder Metallelement mit dem Zahnersatz **8** stoffschlüssig verbunden.

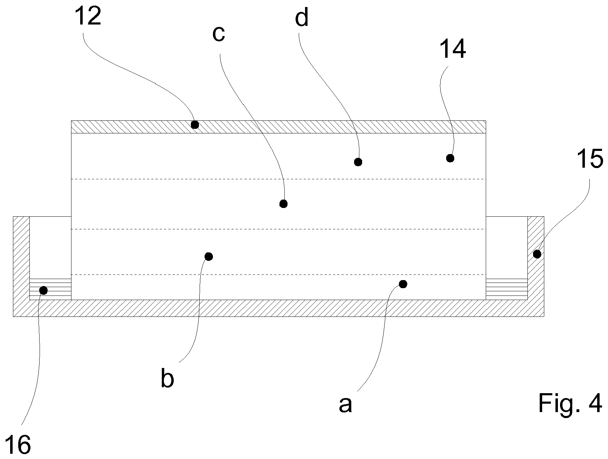

Neben Einfärben von Zahnersatz **8** und/oder Implantate oder Implantatprothetik kann auch ein Keramikblock **14** beziehungsweise eine poröse Keramik vor der CAD/CAM-Bearbeitung eingefärbt werden. In der Fig. 4 ist eine Möglichkeit der Blockeinfärbung dargestellt. Auf die poröse Keramik und/oder Keramikblock **15** ist eine farbgebende Schicht oder Verbundmaterial **12** aufgetragen. In diesem Verbundmaterial **12** sind farbgebende Komponenten und/oder brandfeste Pigmente und/oder Oxide und/oder färbende und fluoreszierende Metalloxide und/oder organische oder anorganische Salze enthalten. Die poröse Keramik und/oder der Keramikblock **14** liegt in einer Schale und/oder Gefäß **15.** In dieser Schale und/oder Gefäß **15** befindet sich eine Flüssigkeit, insbesondere ein Lösungsmittel **16,** um die farbgebenden Komponenten und/oder brandfesten Pigmente und/oder Oxide und/oder färbenden und fluoreszierenden Metalloxide und/oder organischen oder anorganischen Salze aus dem Verbundmaterial **12** herauszulösen und somit eine Infiltration herbeizuführen. Dadurch erhält der Keramikblock beziehungsweise die poröse Keramik **14** Farbabstufungen **a, b, c, d** von hell **a** bis dunkel **d.** Die Farbabstufungen **a, b, c, d** gehen ineinander über und bilden somit einen weichen Übergang, der als gestrichelte Linie dargestellt ist.

### Bezugszeichenliste

1 - poröse und/oder vorgesinterte und/oder entbinderte und/oder gebinderte Keramik bereitstellen
2 - farbgebendes Verbundmaterial auf poröse Keramik auftragen
3 - Wärmebehandlung durchführen, wodurch farbgebende Komponente und/oder brandfeste Pigmente und/oder Oxide und/oder färbenden und fluoreszierenden Metalloxide und/oder organischen oder anorganischen Salze aus dem Verbundmaterial herausgelöst werden
4 - Infiltration der farbgebenden Komponenten und/oder brandfesten Pigmente und/oder Oxide und/oder färbenden und fluoreszierenden Metalloxide und/oder organischen oder anorganischen Salze in die Poren der Keramik
5 - Sintern der Keramik
6 - Verwenden eines Lösungsmittels, um die farbgebenden Komponenten und/oder brandfesten Pigmente und/oder Oxide und/oder färbenden und fluoreszierenden Metalloxide und/oder organischen oder anorganischen Salze aus dem Verbundmaterial herauszulösen
7 - Trocknung der behandelten Keramik
8 - Zahnersatz und/oder Implantatprothetik oder Implantat
9 - Innenfläche des Zahnersatzes und/oder des Implantats oder Implantatsprothetik
10 - Schneide des Zahnersatzes und/oder des Implantats oder Implantatsprothetik
11 - Außenfläche des Zahnersatzes und/oder des Implantats oder Implantatsprothetik
12 - farbgebender Auftrag / Verbundmaterial
13 - Metallgerüst und/oder Metallelement
14 - poröse Keramik und/oder Keramikblock
15- Schale und/oder Gefäß
16- Lösungsmittel / Flüssigkeit
a, b, c, d Einfärbung mit Abstufungen von hell bis dunkel

## Patentansprüche

1. Verfahren zum Färben von gebinderter und/oder entbinderter und/oder angesinterter und/oder durchgängig poröser Keramik (14), insbesondere von porösen Keramikformkörpern, die insbesondere in der Dentaltechnik Anwendung finden, wobei der farbgebende Auftrag mit einem Auftragungs- und/oder stiftartigen Werkzeug erfolgt, welches Werkzeug Verbundmaterial (12), insbesondere Malstiftverbundmaterial aufweist, wobei das Verbundmaterial (12) mit farbgebenden Komponenten und/oder brandfesten Pigmenten und/oder Oxiden und/oder färbenden und fluoreszierenden Metalloxiden und/oder organischen oder anorganischen Salzen versetzt ist,
**dadurch gekennzeichnet, dass**
die farbgebenden Komponenten und/oder brandfesten Pigmente und/oder Oxide und/oder färbenden und fluoreszierenden Metalloxide und/oder organischen oder anorganischen Salze aus dem Verbundmaterial (12) mit einem Lösungsmittel (16) herausgelöst werden, um eine Infiltration herbeizuführen.

2. Verfahren nach Anspruch 1, **dadurch gekennzeichnet, dass** das Verbundmaterial (12) mit den darin beinhalteten Füllstoffen und farbgebenden Komponenten und/oder brandfesten Pigmenten und/oder Oxiden und/oder färbenden und fluoreszierenden Metalloxiden und/oder organischen oder anorganischen Salzen als Folie adaptierbar ist.

3. Verfahren nach einem der vorangehenden Ansprüche, **dadurch gekennzeichnet, dass** als Lösungsmittel (16) Wasser oder eine Mischung aus Wasser mit einem organischen, insbesondere einem polaren organischen, Lösungsmittel (16), verwendet wird.

4. Verfahren nach einem der vorangehenden Ansprüche, **dadurch gekennzeichnet, dass** in das Lösungsmittel (16) ein Oxidationsmittel, insbesondere Wasserstoffperoxid, eingebracht wird.

5. Verfahren nach einem der vorangehenden Ansprüche, **dadurch gekennzeichnet, dass** in das Lösungsmittel (16) Öle und/oder Benzine eingebracht werden.

6. Verfahren nach einem der vorangehenden Ansprüche, **dadurch gekennzeichnet, dass** die farbgebenden Komponenten und/oder brandfesten Pigmente und/oder Oxide und/oder färbenden und fluoreszierenden Metalloxide und/oder organischen oder anorganischen Salze in/unter einer Vakuumatmosphäre herausgelöst werden.

7. Verfahren nach einem der vorangehenden Ansprüche, **dadurch gekennzeichnet, dass** die farbgebenden Komponenten und/oder brandfesten Pigmente und/oder Oxide und/oder färbenden und fluoreszierenden Metalloxide und/oder organischen oder anorganischen Salze unter Druck herausgelöst werden.

8. Verfahren nach einem der vorangehenden Ansprüche, **dadurch gekennzeichnet, dass** Farbmessungen an natürlichen Zähnen in einem programmierbaren Speicher hinterlegt werden, so dass die Farbe der natürlichen Zähne mit dem entsprechenden Verbundmaterial (12) reproduziert werden kann.

9. Verfahren nach einem der vorangehenden Ansprüche, **dadurch gekennzeichnet, dass** eine programmgesteuerte Maschine oder Gerät, insbesondere eine CAD/CAM-Anlage, zum Auftrag oder zum Abtrag eines oder mehrerer Verbundmaterialien (12) entsprechend der festgelegten Zahnfarbe verwendet wird.

10. Verfahren nach Anspruch 9, **dadurch gekennzeichnet, dass** die Farbdaten der durchgeführten Farbmessung an die programmgesteuerte Maschine oder an das programmgesteuerte Gerät übermittelt werden.

11. Verfahren nach einem der vorangehenden Ansprüche, **dadurch gekennzeichnet, dass** ganze vorgesinterte und/oder vorgepresste Keramikblöcke (14) vor der CAD/CAM Bearbeitung eingefärbt werden.

12. Verfahren nach einem der vorangehenden Ansprüche, **dadurch gekennzeichnet, dass** der farbgebende Auftrag oder Verbundmaterial (12) an den Außen- und/oder Innenflächen (9, 11) des Zahnersatzes (8) und/oder der Implantatprothetik oder der Implantate aufgetragen und/oder abgetragen wird.

13. Verfahren nach einem der vorangehenden Ansprüche, **dadurch gekennzeichnet, dass** eine porös gebrannte Dentalverblendkeramik oder eine porös gepresste Glaskeramik als zu bemalende Keramik (14) verwendet wird.

14. Verfahren nach einem der vorangehenden Ansprüche, **dadurch gekennzeichnet, dass** das auf der Keramik (14) aufgetragene und infiltrierte Verbundmaterial (12) unter ansteigender Vakuumatmosphäre oder in einem sauerstofffreien oder annähernd sauerstofffreien Raum gesintert wird, um die farbgebenden Komponenten und/oder brandfesten Pigmente und/oder Oxide und/oder färbenden und fluoreszierenden Metalloxide und/oder organischen oder anorganischen Salze in der Keramik zu fixieren.

15. Verbundmaterial (12), geeignet für Malstifte und zur Verwendung nach einem Verfahren der Ansprüche 1-14, zum Auftragen auf durchgängig poröse Keramik (14), das farbgebende Komponenten und/oder brandfeste Pigmente und/oder Oxide und/oder färbende und fluoreszierende Metalloxide und/oder organische oder anorganische Salze und einen oder mehrere Füllstoffe aufweist, deren Kombination ein Färbemittel, insbesondere für die Zahntechnik, darstellt, wobei das Färbemittel unterschiedliche Konsistenzen aufweist **dadurch gekennzeichnet, dass** die Füllstoffe Nanopartikel, die eine hydrophile Ligandenbeschichtung aufweisen, beinhalten.

## Claims

1. A method for colouring bonded and/or unbonded and/or sintered and/or continuously porous ceramic (14), in particular porous moulded ceramic bodies, which are used in particular in dental technology, the colouring application being carried out using an application and/or pen-like tool, which tool has a composite material (12), in particular a painting pen composite material, the composite material (12) being loaded with colouring components and/or fire-resistant pigments and/or oxides and/or colouring and fluorescent metal oxides and/or organic or inorganic salts,
**characterised in that**
the colouring components and/or fire-resistant pigments and/or oxides and/or colouring and fluorescent metal oxides and/or organic or inorganic salts are dissolved away from the composite material (12) using a solvent (16) in order to bring about infiltration.

2. The method according to Claim 1, **characterised in that** the composite material (12) with the fillers and colouring components and/or fire-resistant pigments and/or oxides and/or colouring and fluorescent metal oxides and/or organic and inorganic salts contained therein can be adapted as film.

3. The method according to any of the preceding claims, **characterised in that** water or a mixture of water with an organic, in particular a polar organic, solvent (16), is used as the solvent (16).

4. The method according to any of the preceding claims, **characterised in that** an oxidising agent, in particular hydrogen peroxide, is introduced into the solvent (16).

5. The method according to any of the preceding claims, **characterised in that** oils and/or benzines are introduced into the solvent (16).

6. The method according to any of the preceding claims, **characterised in that** the colouring components and/or fire-resistant pigments and/or oxides and/or colouring and fluorescent metal oxides and/or organic or inorganic salts are dissolved away in/under a vacuum atmosphere.

7. The method according to any of the preceding claims, **characterised in that** the colouring components and/or fire-resistant pigments and/or oxides and/or colouring and fluorescent metal oxides and/or organic or inorganic salts are dissolved away under pressure.

8. The method according to any of the preceding claims, **characterised in that** colour measurements on natural teeth are stored in a programmable memory so that the colour of the natural teeth can be reproduced with the corresponding composite material (12).

9. The method according to any of the preceding claims, **characterised in that** a programme-controlled machine or device, in particular a CAD/CAM system, is used to apply or to remove one or more composite materials (12) according to the tooth colour that has been determined.

10. The method according to Claim 9, **characterised in that** the colour data of the colour measurement that is taken are passed to the programme-controlled machine or to the programme-controlled device.

11. The method according to any of the preceding claims, **characterised in that** entire pre-sintered and/or pre-pressed ceramic blocks (14) are coloured before the CAD/CAM processing.

12. The method according to any of the preceding claims, **characterised in that** the colouring application or composite material (12) is applied to and/or removed from the outer and/or inner surfaces (9, 11) of the denture (8) and/or the implant prosthetic or the implants.

13. The method according to any of the preceding claims, **characterised in that** a porously burnt dental veneer ceramic or a porously pressed glass ceramic is used as the ceramic (14) to be painted.

14. The method according to any of the preceding claims, **characterised in that** the composite material (12) applied and infiltrated onto the ceramic (14) is sintered in an increasing vacuum atmosphere or in an oxygen-free or almost oxygen-free space in order to fix the colouring components and/or fire-resistant pigments and/or oxides and/or colouring and fluorescent metal oxides and/or organic or inorganic salts in the ceramic.

15. A composite material (12), suitable for painting pens and for use according to a method of Claims 1-14, for applying to continuously porous ceramic (14), and that has colouring components and/or fire-resistant pigments and/or oxides and/or colouring and fluorescent metal oxides and/or organic or inorganic salts and one or more fillers, the combination of which constitutes a colorant, in particular for dental technology, the colourant having different consistencies, **characterised in that** the fillers contain nanoparticles which have a hydrophilic ligand coating.

## Revendications

1. Procédé pour colorer une céramique (14) liée et/ou non liée et/ou frittée et/ou entièrement poreuse, en particulier des corps de forme en céramique poreuse, qui sont utilisées en particulier dans la technique dentaire, dans lequel l'application réalisant la couleur est mise en oeuvre en utilisant un outil de revêtement et/ou en forme de tige, lequel outil comprend un matériau composite (12), en particulier un matériau composite d'application de peinture, dans lequel le matériau composite (12) est chargé avec des composants de coloration et/ou des pigments résistants au feu et/ou des oxydes et/ou des oxydes métalliques colorants et fluorescents et/ou des sels organiques ou non organiques, **caractérisé en ce que**
les composants de coloration et/ou les pigments résistants au feu et/ou les oxydes et/ou les oxydes métalliques colorants et fluorescents et/ou les sels organiques ou non organiques sont extraits du matériau composite (12) avec un solvant (16), pour provoquer une infiltration.

2. Procédé selon la revendication 1, **caractérisé en ce que** le matériau composite (12) avec les charges qu'il contient et les composants de coloration et/ou les pigments résistants au feu et/ou les oxydes et/ou les oxydes métalliques colorants et fluorescents et/ou les sels organiques ou non organiques peut être conformé sous forme de film.

3. Procédé selon l'une quelconque des revendications précédentes, **caractérisé en ce que**, en tant que solvant (16) on utilise de l'eau ou un mélange d'eau avec un solvant organique (16), en particulier un solvant organique polaire.

4. Procédé selon l'une quelconque des revendications précédentes, **caractérisé en ce que** dans le solvant (16) on ajoute un oxydant, en particulier du peroxyde d'hydrogène.

5. Procédé selon l'une quelconque des revendications précédentes, **caractérisé en ce qu'**on ajoute dans le solvant (16) des huiles et/ou des essences.

6. Procédé selon l'une quelconque des revendications précédentes, **caractérisé en ce que** les composants de coloration et/ou les pigments résistants au feu et/ou les oxydes et/ou les oxydes métalliques colorants et fluorescents et/ou les sels organiques ou non organiques sont extraits dans/sous une atmosphère de vide.

7. Procédé selon l'une quelconque des revendications précédentes, **caractérisé en ce que** les composants de coloration et/ou les pigments résistants au feu et/ou les oxydes et/ou les oxydes métalliques colorants et fluorescents et/ou les sels organiques ou non organiques sont extraits sous pression.

8. Procédé selon l'une quelconque des revendications précédentes, **caractérisé en ce que** des mesures de couleur sur des dents naturelles sont enregistrées dans une mémoire programmable, de façon que la couleur des dents naturelles puisse être reproduite avec le matériau composite (12) approprié.

9. Procédé selon l'une quelconque des revendications précédentes, **caractérisé en ce qu'**on utilise une machine commandée par un programme ou un dispositif commandé par un programme, en particulier un système CAD/CAM, pour appliquer ou pour enlever un ou plusieurs matériaux composites (12) en fonction de la couleur particulière de la dent.

10. Procédé selon la revendication 9, **caractérisé en ce que** les données de couleur des mesures de couleurs qui ont été effectuées sont transmises à la machine commandée par programme ou au dispositif commandé par programme.

11. Procédé selon l'une quelconque des revendications précédentes, **caractérisé en ce que** la totalité des blocs de céramique (14) préalablement frittés et/ou préalablement comprimés sont colorés avant le traitement CAD/CAM.

12. Procédé selon l'une quelconque des revendications précédentes, **caractérisé en ce que** la coloration ou le matériau composite (12) sont appliqués sur et/ou sont retirés des surfaces extérieures et/ou intérieures (9, 11) de la prothèse dentaire (8) et/ou de la prothèse implantable ou de l'implant.

13. Procédé selon l'une quelconque des revendications précédentes, **caractérisé en ce qu'**on utilise comme céramique (14) à colorer une céramique dentaire poreuse étirée ou une céramique vitreuse poreuse pressée.

14. Procédé selon l'une quelconque des revendications précédentes, **caractérisé en ce que** le matériau composite (12) appliqué sur et infiltré dans la céramique (14) est fritté sous une atmosphère à vide croissant ou dans un espace dépourvu d'oxygène ou presque dépourvu d'oxygène de façon à fixer dans la céramique les composants de coloration et/ou les pigments résistants au feu et/ou les oxydes et/ou les oxydes métalliques colorants et fluorescents et/ou les sels organiques ou non organiques.

15. Matériau composite (12), adapté pour des tiges et pour la mise en oeuvre d'un procédé selon les revendications 1 à 14, pour appliquer sur une céramique (14) entièrement poreuse les composants de coloration et/ou les pigments résistants au feu et/ou les oxydes et/ou les oxydes métalliques colorants et fluorescents et/ou les sels organiques ou non organiques et une ou plusieurs charges, dont la combinaison constitue un matériau colorant, en particulier pour la technique dentaire, dans lequel le matériau colorant présente différentes consistances, **caractérisé en ce que** les charges comportent des nanoparticules qui comportent un revêtement en un ligand hydrophile.
